# EUROPEAN PATENT SPECIFICATION

(11) **EP 2 448 489 B1**
(45) Date of publication and mention of the grant of the patent: **27.11.2013**
(21) Application number: 10730956.9
(22) Date of filing: 01.07.2010
(51) Int. Cl.: A61B 5/151

(54) **LANCET**
LANZETTE
LANCETTE

(30) Priority: 02.07.2009 US 222567 P
(43) Date of publication of application: 09.05.2012
(73) Proprietor: Facet Technologies, LLC, Kennesaw, GA 30144 (US)
(72) Inventor: TRISSEL, John, Canton GA 30115 (US); PUSEY, Lauren, R., Woodstock GA 30188 (US); RUF, Christopher, J., Sandy Springs, GA 30328, (US)
(74) Representative: McCartney, Jonathan William
(86) International application number: PCT/US2010/040794
(87) International publication number: WO 2011/003001

(56) References cited:
- EP-A1- 1 987 770
- WO-A1-2009/006461
- WO-A2-2007/086843
- WO-A2-2009/015097
- DE-U1- 20 114 658
- US-A1- 2003 109 895
- US-A1- 2007 162 064
- US-A1- 2010 030 249
- US-S1- D 484 244

## Description

### Technical Field

The present invention relates generally to the field of medical devices, and more particularly to lancets and lancing devices for blood sampling and testing.

### Background

Lancets and lancing devices are utilized for penetrating the skin of a human or animal subject at a lancing site to obtain a sample of blood or other body fluid for medical testing, as in blood-typing or blood-glucose testing. Known lancing devices commonly include a housing containing a drive mechanism, a charging mechanism for energizing the spring or other drive means of the drive mechanism, and a release mechanism for releasing the drive mechanism upon actuation. A lancet is typically propelled by the drive mechanism from a retracted position shielded within the housing to an extended position wherein a sharp tip portion of the lancet projects from the housing to prick the subject's skin at a desired lancing site. An ejection mechanism can optionally be included for discharge of a used lancet from the lancing device.

The lancet is typically a disposable component that is removably mounted into a receiver or lancet carrier portion of the drive mechanism of a lancing device. A used lancet typically is removed from the lancet carrier after sampling for disposal. A new, sterile lancet is then replaced into the lancet carrier for further sampling. Lancets typically comprise a sharp metal tip in the form of a needle or blade. The needle or blade is typically embedded in a plastic body that has a size and shape configured for releasable engagement with the receiver or lancet carrier of a lancing device. The sharp tip of the lancet is commonly embedded in a removable plastic cap to maintain sterility and prevent inadvertent sticks prior to use. The endcap may be replaceable onto the lancet after use to re-cover the sharp lancet tip for safety and hygienic purposes. It is to the provision of an improved lancet that the present invention is primarily directed.

US2003/0109895 discloses a lancet having a coupling profile that is compatible with lancet holding features of standard lancing devices, but is keyed for mounting in a non-standard lancing devices having a cooperating keyed coupled profile.

WO2009/015097 discloses a lancing system comprising a lancet with handle portion and a needle bearing portion, the needle bearing portion retaining a thin needle protected by a safety cap. The safety cap includes first and second chambers where the first chamber acts as sheath and the second chamber having a surface positioned to compromise the structure of the needle when the needle bearing portion of the lancet body is inserted into the second chamber.

WO2007/086843 discloses a blood glucose measuring system including a lancet sensor assembly and a meter for use with the lancet sensor assembly. The lancet sensor assembly has a lancet member with a lance, a lancet body having a drive wing extending outwardly from a side, and a sinuous portion, and an elongated carrier having a lancet member recess to contain the lancet member, a open end, a closed end, a side elongated opening for receiving the drive wing therethrough, and an anchoring member operatively connected to the end of the sinuous portion.

US2007/0162064 discloses an improved protective lancet, a lancet casing and a method for producing the same. The lancet casing includes a lancet body that is integrally molded about an elongated shaft portion of a lancet needle. The lancet body is characterized in having an upper bearing surface from which a pointed end of the lancet needle extends. The lancet casing further includes a cap integrally molded about the pointed end of the lancet needle and joined to the lancet body by a narrow frangible junction on the upper bearing surface of the lancet body. In accordance with the present invention, one or more tab members are affixed between the cap and the body. Each of the one or more tab members includes a persistently affixed upper end integrally molded to the outer contour of the cap and a detachably affixed lower end integrally molded to or near a peripheral edge of the upper bearing surface of the lancet body to provide a readily perceptible user indication of the status of whether or not the cap member has been reattached or tampered with.

The invention is defined in the attached independent claim to which reference should now be made. Further, optional features may be found in the sub-claims appended thereto.

### Summary

In example embodiments, the present invention relates to a proprietary lancet configured for insertion into a cooperating receiver of a lancing device. The lancet and lancing device include complementary engagement or coupling features for ensuring that only the proprietary lancet design is used with the lancing device. The engagement features provide a consistent lancet placement in the receiver, maintaining a consistent penetration depth and proper alignment for pain management.

The lancet optionally includes a fine gauge lancet needle, for example a 33-gauge needle. The lancet optionally also includes one or more relief channels in a removable endcap portion surrounding a sheath for retaining the lancet needle. The mold tooling that forms the relief channels directs the flow of plastic during injection molding of the lancet to reduce bending forces on the tip of the lancet needle during the molding process.

The features and advantages of the invention will be understood with reference to the drawing figures and detailed description herein, and will be realized by means of the various elements and combinations particularly pointed out in the appended claims. It is to be understood that both the foregoing general description and the following brief description of the drawings and detailed description of the invention are exemplary and explanatory of preferred embodiments of the invention, and are not restrictive of the invention, as claimed.

### Brief Description of the Drawings

**FIGURE 1** is a first perspective view of a lancet according to an example embodiment of the present invention.

**FIGURE 2** is a second perspective view of the lancet of Figure 1.

**FIGURE 3** is a perspective view of the lancet of Figure 1, with an endcap portion thereof removed to expose the lancet needle.

FIGURE 4 is a rear end view of the lancet of Figure 1.

### Detailed Description of Example Embodiments

The present invention may be understood more readily by reference to the following detailed description of the invention taken in connection with the accompanying drawing figures, which form a part of this disclosure.

Also, as used in the specification including the appended claims, the singular forms "a," "an," and "the" include the plural, and reference to a particular numerical value includes at least that particular value, unless the context clearly dictates otherwise. Ranges may be expressed herein as from "about" or "approximately" one particular value and/or to "about" or "approximately" another particular value. When such a range is expressed, another embodiment includes from the one particular value and/or to the other particular value. Similarly, when values are expressed as approximations, by use of the antecedent "about," it will be understood that the particular value forms another embodiment.

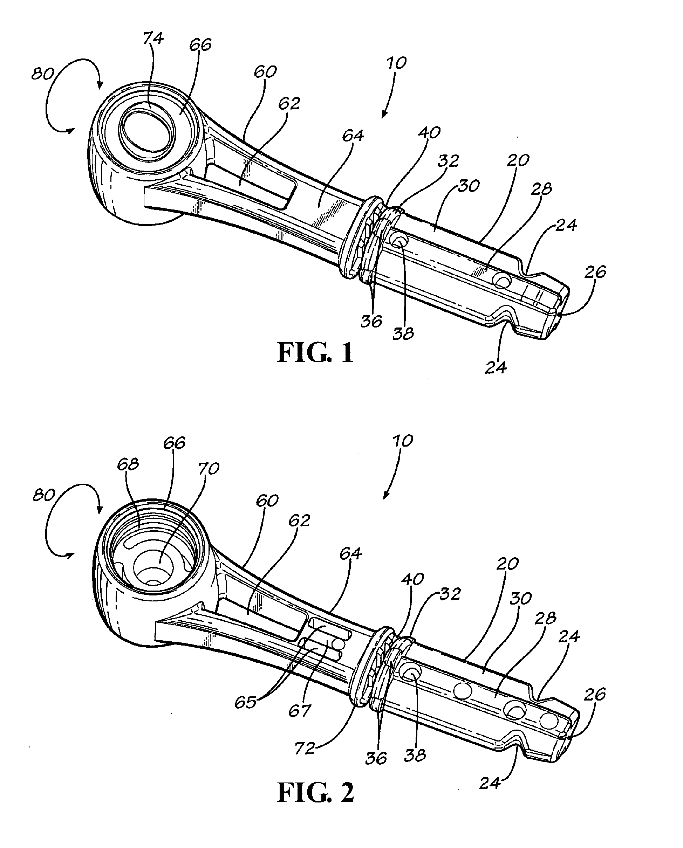

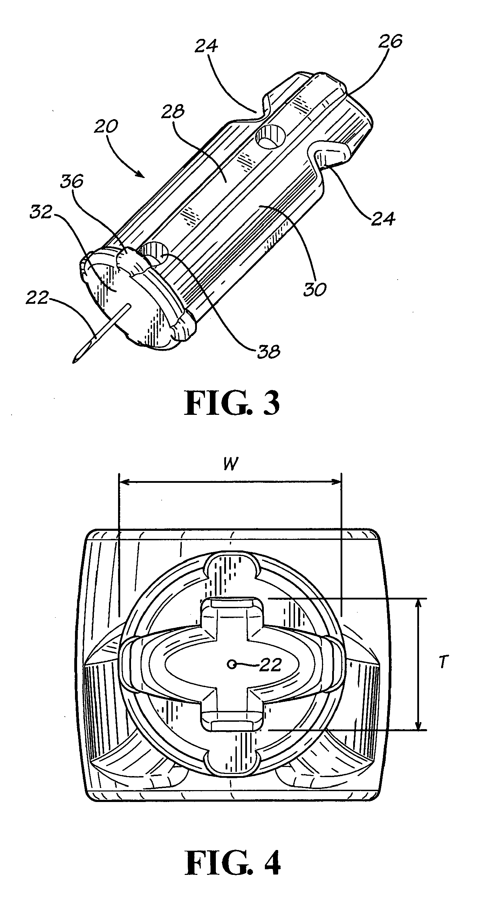

With reference now to the drawing figures, wherein like reference numbers represent corresponding parts throughout the several views, Figures 1 and 2 show a proprietary interchangeable lancet 10, which includes lancet body 20, one or more breakaway connections or attachment webs 40, and an endcap 60. Figure 3 shows the lancet 20 with the endcap 60 removed. In example embodiments, the lancet 10 has engagement features that are unique and complementary to a specified lancing device, so that only lancets of the proprietary design are usable with the lancing device, and preventing use of the lancing device with unauthorized lancets which may not be of adequate quality or safety, or which may lack the proper characteristics specified by the lancing device manufacturer or healthcare provider for a given application.

The lancet body 20 includes a needle 22, a rear end 26 for releasable engagement within a cooperating receiver or lancet holder portion of a corresponding lancing device design, a first pair of longitudinal protrusions or ribs 28 extending along at least a portion of its length on opposed top and bottom faces, a second pair of longitudinal protrusions or fins 30 extending along at least a portion of opposed side faces of the lancet body and arranged generally crosswise to the first pair of longitudinal protrusions or ribs 28, and a front end forming a transverse collar or flange 32. The lancet body 20 has a generally elliptical shaped periphery when viewed end-on or in cross-section, with the ribs 28 constituting the opposing ends of the short axis of the elliptical periphery and the fins 30 constituting the opposing ends of the long axis of the elliptical periphery. Other cross-sectional geometries are within the scope of the invention, including geometries having or being circumscribable within peripheral shapes including non-circular, rectangular, polygonal, square, triangular, circular and/or keyed. The ribs 28 and the fins 30 taper inwardly along their longitudinal axes towards the rear end 26. Along the sides of the fins 30 are formed an opposed pair of releasable engagement recesses 24 that are located proximally to the rear end. The front flange or collar 32 has a generally circular profile, with rounded protrusions 36 spaced crosswise along its perimeter. The lancet 20 optionally defines one or more recesses or holes 38, formed by mold tooling during manufacture for holding the lancet needle in position in the mold, and which reduce the amount of material required to manufacture the lancet.

The needle 22 includes a sharp tip portion extending axially from the lancet body 20 opposite the rear end 26. The needle can have a round cross-section, or can comprise a flat blade, or can be otherwise configured. Optionally, the needle 22 extends axially through the entire length of the lancet body, and a distal end of the needle is exposed at or extends beyond the rear end of the lancet body. Alternatively, the distal end of the needle is embedded within the lancet body. In example embodiments, the needle has a fine gauge, for example a 33-gauge needle, for lower mass and reduced pain during use.

The endcap 60 includes a body 64, a receiver cup or receptacle 66 for attachment over the lancet needle after use, and an end disk or flange 72. The body 64 tapers outwards along its longitudinal axis from the end disk 72 towards the receptacle 66. The end disk 72 contains a needle opening that receives the lancet needle 22 in the lancet's unused state. The endcap 60 may include branding indicia 74 or other information about the product on the receptacle 66 or elsewhere on the endcap and/or lancet body. The body may contain one or more material voids 62 to lessen the amount of material used to construct the needle cap. The end disk 72 has a generally circular cross section. The receptacle 66 contains an inner groove or snap coupling 68 and a central chamber or opening 70. The inner groove 68 releasably engages the round protrusions 36 of the lancet body to retain the lancet needle shielded within the cup 66 after use of the lancet to prevent accidental needle sticks. The central chamber or opening 70 receives the needle tip after use of the lancet to prevent breaking the tip and to provide easier attachment of the endcap onto the lancet body. In example embodiments of the invention, the depth of the chamber 70 is equal to or greater than the length of the exposed portion of the needle 22 extending from the lancet body.

The lancet 20 is connected to the lancet endcap 60 by one or more breakaway connections or webs 40. The breakaway connections or webs are thin bodies or protrusions that extend between the front disk 34 of the lancet body and the end disk 72 of the endcap that may be broken or separated by applying a torsional (indicated by direction arrow 80) and/or tension force to the needle cap 60 while holding the lancet body. The breakaway connections or webs 40 may be at least partially severed after molding to provide easier cap removal or may be molded in such a fashion that they are in close proximity to the opposing surface but not connect to it except in the center near the needle. The breakaway connections or webs 40 also serve to provide lateral support to resist bending between the lancet body and the endcap, which might otherwise result in bending of the lancet needle which could result in increased pain to the subject during lancing due to non-axial alignment of the needle tip.

The endcap 60 optionally includes a pair of relief openings, channels, or recesses 65 extending along a portion of the endcap body 64 proximal the end disk or flange 72. During the molding process, tool inserts of the mold that form these recesses 65 channel the flow of plastic to form a sheath 67 extending between the recesses that surrounds the tip of the lancet needle 22. Controllably directing the flow of plastic during molding in this manner reduces or eliminates the application of non-axial bending forces on the lancet needle during manufacture, providing a more straight and axially aligned needle tip and thereby reducing pain during use of the lancet. The relief openings 65 may also permit flexure of the sheath 67 during removal of the endcap to further reduce the imposition of bending forces on the needle tip. In example embodiments, the sheath is formed of a sufficiently deformable resilient material and the thickness of the sheath and the dimensions of the relief openings are selected to result in a sheath stiffness that is less than the stiffness of the lancet needle, such that the sheath bends before the needle. For example, the relief openings may have a length of at least about one-third to one-half the length of the exposed portion of the needle, and/or the width of the relief openings is at least about 0.5 to 1.0 times the thickness of the sheath.

The endcap 60 is optionally at least as long as the lancet body 20, to provide easier manipulation and use of the lancet. In example embodiments, the receiver cup 66 of the endcap has a transverse dimension or diameter that is substantially greater than the width or thickness of the body portion 64 of the endcap, optionally comprising a smoothly tapered or flared transition portion extending therebetween, for gripping during use of the lancet. In the depicted embodiment, the ribs 28 and fins 30 extend along substantially the entire length of the lancet body. In alternate embodiments, the ribs 28 and/or the fins 30 comprise one or more protrusions or bosses extending along a portion of the length of the lancet body, for example positioned at spaced apart locations on the lancet body.

In example forms, and as seen with reference to Figure 4, the rear end 26 of the lancet body 20 presents a non-circular periphery having a width-to-thickness aspect ratio of about 3:2, or of greater than 3 to 2. In other words, the side-to-side width W of the opposed fins 30 is about one and one-half times the top-to-bottom thickness T of the opposed ribs 28. In example forms, the lancet body (the overall lancet 10 with the endcap 60 removed) has a mass of about 0.15 g or less, and more preferably a mass of no more than 0.13 g, thereby providing a low-mass lancet which may reduce the sensation of pain during use. In example forms, the overall length of the lancet body and needle, from the rear end 26 of the body to the tip of the needle 22, is about 16.5 mm, and the length of the lancet body without the needle is about 13 mm.

In use, the lancet 10 is installed into a lancet carrier of a lancing device, rear end 26 of the lancet body 20 first, until the engagement recesses 24 are engaged with cooperating cantilevers or other releasable engagement structures of the lancing device. Engagement of the lancet into the lancing device optionally provides tactile and/or audible feedback to the user to indicate that the engagement structures of the lancing device are fully engaged inside the lancet's engagement recesses. The user then detaches the endcap 60 from the lancet body 20 by separating the breakaway connection webs 40. The non-circular profile of the portion of the lancet body engaged with the lancet carrier prevents the lancet 20 from rotating as the endcap 60 is twisted for removal. The endcap 60 is then axially removed from the lancet needle 22 to expose the sharp tip. The lancing device is charged or cocked, and is then ready for actuation to pierce the subject's skin at the lancing site to generate a sample. The ribs 28 and fins 30 assist in maintaining alignment of the lancet during the lancing stroke, to ensure axial alignment of the lancing needle as it pierces the skin in order to minimize pain. Once the lancet 20 has been used, the lancet needle 22 can be re-covered by placement of the endcap receptacle 66 over the sharp tip of the lancet needle and engaging the protrusions 26 of the lancet body onto to the inner groove 68 of the endcap. The body 64 of the endcap may be used as a handle for the lancet needle cap, and may allow easier re-covering of the lancet needle 22.

While the invention has been described with reference to preferred and example embodiments, it will be understood by those skilled in the art that a variety of modifications, additions and deletions are within the scope of the invention, as defined by the following claims.

## Claims

1. A lancet (10) comprising:
a lancet body (20) having a rear end (26) defining a coupling portion for connection to a lancing device, the coupling portion having a non-circular profile and at least one engagement feature for releasable engagement with a cooperating portion of the lancing device;
a needle (22) having a sharp tip portion extending axially from the lancet body opposite the rear end (26); and
an endcap (60) removably secured to the lancet body and covering the sharp tip portion of the needle (22);
**characterized in that**:
the lancet body (20) includes a first pair of longitudinal ribs (28) extending along at least a portion of its length on opposed top and bottom faces, and a second pair of longitudinal fins (30) extending along at least a portion of opposed side faces and arranged generally crosswise to the first pair of longitudinal ribs (28);
wherein the at least one engagement feature comprises an opposed pair of releasable engagement recesses (24) formed in the sides of the fins (30) and located proximally to the rear end (26);
wherein the non-circular profile of the coupling portion of the lancet body has a width W and a thickness T defining an aspect ratio (W:T) of about 3:2 or greater; and
wherein the lancet body (20) has a generally elliptical shaped periphery when viewed end-on, with the ribs (28) constituting the opposing ends of the short axis of the elliptical periphery and the fins (30) constituting the opposing ends of the long axis of the elliptical periphery.

2. The lancet of claim 1, wherein the endcap (60) comprises a receptacle (66) for enclosing the sharp tip portion of the needle (22) after the lancet is used.

3. The lancet of claim 2, wherein the endcap (60) has a proximal end and a distal end, the proximal end of the endcap (60) being initially secured to the lancet body (20) and covering the sharp tip portion of the needle (22), and the distal end of the endcap (60) comprising the receptacle (66) for enclosing the sharp tip portion of the needle (22) after the lancet is used.

4. The lancet of claim 1, wherein the endcap (60) has relief cutouts (65) alongside a sheath (67) adjacent its proximal end, the sheath initially surrounding the sharp tip portion of the needle.

5. The lancet of claim 1, wherein the lancet body and the needle have a combined mass of no more than 0.15 g and/or have a combined length of about 16.5 mm and/or wherein the lancet body has a length of about 13 mm.

6. The lancet of claim 1, in combination with a lancing device comprising the cooperating portion for releasable engagement with the lancet (10), the engagement feature of the lancet (10) and the cooperating portion of the lancing device being complementary and unique to prevent use of the lancing device with other lancet types.

7. The lancet of claim 1, wherein the opposed pair of recesses (24) are directed inwardly into the lancet body (20).

8. The lancet of claim 7, wherein the inwardly directed recesses (24) are generally V-shaped, having angularly inclined forward and rear contact faces.

9. The lancet of claim 1, wherein the endcap (60) further comprises a receptacle (66) having a depth sufficient to receive the sharp tip portion of the needle (22).

10. The lancet of claim 1, wherein the endcap (60) has relief cutouts (65) alongside a sheath portion (67) into which the sharp tip portion of the needle (22) is embedded.

11. The lancet of claim 1, wherein the sharp tip portion of the needle (22) is shielded within a sheath portion (67) of the removable endcap (60), and wherein a pair of relief openings (65) are defined within the removable endcap (60) on opposite sides of the sheath portion (67).

12. The lancet of claim 11, wherein the relief openings (65) provide a sufficient degree of flexure to the sheath portion (67) to resist bending of the sharp tip portion of the needle (22).

13. The lancet of claim 11, wherein the removable endcap (60) further comprises a receptacle (66) distal the sheath portion (67) for receiving the sharp tip portion of the needle (22), and wherein the receptacle (66) may comprise a chamber (70) having a depth at least equal to a length of the sharp tip portion of the needle (22).

14. The lancet of claim 1, wherein the pair of longitudinal ribs (28) and the pair of longitudinal fins (30) taper inwardly along their longitudinal axes towards the rear end (26).

## Patentansprüche

1. Lanzette (10), umfassend:
einen Lanzettenkörper (20), dessen hinteres Ende (26) einen Kopplungsteil zur Verbindung mit einer Stechvorrichtung definiert, wobei der Kopplungsteil ein nicht kreisförmiges Profil und mindestens eine Einrastfunktion zum lösbaren Einrasten mit einem zusammenwirkenden Teil der Stechvorrichtung aufweist;
eine Nadel (22), deren zugespitzter Teil axial vom Lanzettenkörper gegenüber dem hinteren Ende (26) ausgeht; und
eine Endkappe (60), die abnehmbar am Lanzettenkörper befestigt ist und die den zugespitzten Teil der Nadel (22) abdeckt;
**dadurch gekennzeichnet, dass**:
der Lanzettenkörper (20) ein erstes Paar länglicher Rippen (28) aufweist, die längs von mindestens einem Teil seiner Länge auf gegenüberliegenden oberen und unteren Seiten verlaufen, und ein zweites Paar länglicher Flügel (30), die längs von mindestens einem Teil der gegenüberliegenden Seitenflächen verlaufen und im Allgemeinen quer zum ersten Paar länglicher Rippen (28) angeordnet sind;
wobei die mindestens eine Einrastfunktion ein gegenüber liegendes Paar lösbarer Einrastkerben (24) umfasst, die in den Seiten der Flügel (30) herausgearbeitet sind und die sich proximal dem hinteren Ende (26) befinden;
wobei das nicht kreisförmige Profil des Kopplungsteils des Lanzettenkörpers eine Breite W und eine Dicke T aufweist, welche ein Seitenverhältnis (W:T) von etwa 3:2 oder größer definieren; und
wobei der Lanzettenkörper (20) von der Endaufsicht her gesehen einen im Allgemeinen elliptisch geformten Randbereich hat, wobei die Rippen (28) die gegenüberliegenden Enden der kurzen Achse des elliptischen Randbereichs ausmachen und die Flügel (30) die gegenüberliegenden Enden der langen Achse des elliptischen Randbereichs ausmachen.

2. Lanzette nach Anspruch 1, wobei die Endkappe (60) ein Aufnahmefach (66) zum Aufnehmen des zugespitzten Teils (22) nach dem Gebrauch der Lanzette umfasst.

3. Lanzette nach Anspruch 2, wobei die Endkappe (60) ein proximales Ende und ein distales Ende aufweist, das proximale Ende der Endkappe (60) zu Beginn am Lanzettenkörper (20) befestigt ist und den zugespitzten Teil der Nadel (22) aufnimmt, und das distale Ende der Endkappe das Aufnahmefach (66) zum Aufnehmen des zugespitzten Teils der Nadel (22) nach dem Gebrauch der Lanzette umfasst.

4. Lanzette nach Anspruch 1, wobei die Endkappe (60) Entlastungsausschnitte (65) neben einer Hülle (67) nächst ihrem proximalen Ende aufweist, und die Hülle zu Beginn den zugespitzten Teil der Nadel umgibt.

5. Lanzette nach Anspruch 1, wobei der Lanzettenkörper und die Nadel eine Gesamt-Masse von nicht mehr als 0,15 g haben und/oder eine Gesamt-Länge von etwa 16,5 mm haben und/oder wobei der Lanzettenkörper eine Länge von etwa 13 mm aufweist.

6. Lanzette nach Anspruch 1, in Kombination mit einer Stechvorrichtung, umfassend den zusammenwirkenden Teil zum lösbaren Einrasten mit der Lanzette (10), wobei die Einrastfunktion der Lanzette (10) und der zusammenwirkende Teil der Stechvorrichtung komplementär und einzigartig sind, damit die Verwendung der Stechvorrichtung mit anderen Lanzettentypen verhindert wird.

7. Lanzette nach Anspruch 1, wobei das gegenüberliegende Paar Kerben (24) einwärts in den Lanzettenkörper (20) gerichtet sind.

8. Lanzette nach Anspruch 7, wobei die einwärts gerichteten Kerben (24) im Allgemeinen V-förmig sind, und sie angewinkelte Vorder- und Rück-Kontaktseiten haben.

9. Lanzette nach Anspruch 1, wobei die Endkappe (60) zudem ein Aufnahmefach (66) umfasst, dessen Tiefe hinreicht, dass der zugespitzte Teil der Nadel (22) aufgenommen wird.

10. Lanzette nach Anspruch 1, wobei die Endkappe (60) EntlastungsAusschnitte (65) neben einem Hüllenteil (67) hat, in den der zugespitzte Teil der Nadel (22) eingebettet ist.

11. Lanzette nach Anspruch 1, wobei der zugespitzte Teil der Nadel (22) mit einem Hüllenteil (67) der abnehmbaren Endkappe (60) ummantelt ist, und wobei ein Paar Entlastungsöffnungen (65) in der abnehmbaren Endkappe (60) auf gegenüberliegenden Seiten des Hüllenteils (67) definiert ist.

12. Lanzette nach Anspruch 11, wobei die Entlastungsöffnungen (65) dem Hüllenteil (67) einen hinreichenden Grad an Biegsamkeit verleihen, dass einem Verbiegen des zugespitzten Teils der Nadel (22) standgehalten wird.

13. Lanzette nach Anspruch 11, wobei die abnehmbare Endkappe (60) zudem ein Aufnahmefach (66) distal des Hüllenteils (67) zum Aufnehmen des zugespitzten Teils der Nadel (22) aufweist, und wobei das Aufnahmefach (66) eine Kammer (70) umfassen kann, deren Tiefe mindestens gleich einer Länge des zugespitzten Teils der Nadel (22) ist.

14. Lanzette nach Anspruch 1, wobei sich das Paar längliche Rippen (28) und das Paar längliche Flügel (30) längs ihrer Längsachsen zum Hinterende (26) nach innen verjüngen.

## Revendications

1. Lancette (10) comprenant :
un corps de lancette (20) ayant une extrémité arrière (26) définissant une portion de couplage pour la connexion à un dispositif de prélèvement , la portion de couplage ayant un profil non circulaire et au moins une propriété d'engagement pour une mise en prise libérable avec une portion de coopération du dispositif de prélèvement ;
une aiguille (22) ayant une portion de pointe affûtée s'étendant axialement du corps de lancette opposée à l'extrémité arrière (26) ; et
un capuchon d'extrémité (60) fixé d'une manière amovible au corps de lancette et couvrant la portion de pointe affûtée de l'aiguille (22) ;
**caractérisée en ce que** :
le corps de lancette (20) comprend une première paire de nervures longitudinales (28) s'étendant le long d'au moins une portion de sa longueur sur des faces supérieure et inférieure opposées, et une seconde paire d'ailettes longitudinales (30) s'étendant le long d'au moins une portion de faces latérales opposées et agencées généralement de façon transversale à la première paire de nervures longitudinales (28) ;
où la au moins une propriété d'engagement comprend une paire d'évidements d'engagement libérable opposés (24) ménagés dans les côtés des ailettes (30) et situées proximalement à l'extrémité arrière (26) ;
où le profil non circulaire de la portion de couplage du corps de lancette a une largueur W et une épaisseur T définissant un rapport d'aspect (W:T) d'environ 3:2 ou plus grand ; et
où le corps de lancette (20) possède une périphérie de forme généralement elliptique lorsqu'il est vu avec l'extrémité en place, les nervures (28) constituant les extrémités opposées de l'axe court de la périphérie elliptique et les ailettes (30) constituant les extrémités opposées de l'axe long de la périphérie elliptique.

2. Lancette selon la revendication 1, où le capuchon d'extrémité (60) comprend un réceptacle (66) pour renfermer la portion de pointe affûtée de l'aiguille (22) après que la lancette a été utilisée.

3. Lancette selon la revendication 2, où le capuchon d'extrémité (60) possède une extrémité proximale et une extrémité distale, l'extrémité proximale du capuchon d'extrémité (60) étant initialement fixée au corps de lancette (20) et couvrant la portion de pointe affûtée de l'aiguille (22), et l'extrémité distale du capuchon d'extrémité (60) comprenant le réceptacle (66) pour renfermer la portion de pointe affûtée de l'aiguille (22) après que la lancette a été utilisée.

4. Lancette selon la revendication 1, où le capuchon d'extrémité (60) possède des découpures de dégagement (65) le long d'une gaine (67) adjacente à son extrémité proximale, la gaine entourant initialement la portion de pointe affûtée de l'aiguille.

5. Lancette selon la revendication 1, où le corps de lancette et l'aiguille ont une masse combinée non supérieure à 0,15 g et/ou ont une longueur combinée d'environ 16,5 mm et/ou où le corps de lancette a une longueur d'environ 13 mm.

6. Lancette selon la revendication 1, en combinaison avec un dispositif de prélèvement comprenant la portion de coopération pour la mise en prise libérable avec la lancette (10), la propriété d'engagement de la lancette (10) et de la portion de coopération du dispositif de prélèvement étant complémentaire et unique pour prévenir l'utilisation du dispositif de prélèvement avec d'autres types de lancettes.

7. Lancette selon la revendication 1, où la paire d'évidements opposés (24) sont dirigés vers l'intérieur dans le corps de lancette (20).

8. Lancette selon la revendication 7, où les évidements dirigés vers l'intérieur (24) sont généralement en forme de V, ayant des faces de contact avant et arrière angulairement inclinés.

9. Lancette selon la revendication 1, où le capuchon d'extrémité (60) comprend en outre un réceptacle (66) ayant une profondeur suffisante pour recevoir la portion de pointe affûtée de l'aiguille (22).

10. Lancette selon la revendication 1, où le capuchon d'extrémité (60) possède des découpures de dégagement (65) le long d'une portion de gaine (67) dans laquelle la portion de pointe affûtée de l'aiguille (22) est noyée.

11. Lancette selon la revendication 1, où la portion de pointe affûtée de l'aiguille (22) est protégée dans une portion de gaine (67) du capuchon d'extrémité amovible (60), et où une paire d'ouvertures de dégagement (65) sont définies dans le capuchon d'extrémité amovible (60) sur les côtés opposés de la portion de gaine (67).

12. Lancette selon la revendication 11, dans laquelle les ouvertures de dégagement (65) fournissent un degré suffisant de flexion à la portion de gaine (67) pour résister à la déformation de la portion de pointe affûtée de l'aiguille (22).

13. Lancette selon la revendication 11, où le capuchon d'extrémité amovible (60) comprend en outre un réceptacle (66) distal à la portion de gaine (67) pour recevoir la portion de pointe affûtée de l'aiguille (22), et où le réceptacle (66) peut comprendre une chambre (70) ayant une profondeur au moins égale à une longueur de la portion de pointe affûtée de l'aiguille (22).

14. Lancette selon la revendication 1, où la paire de nervures longitudinales (28) et la paire d'ailettes longitudinales (30) diminuent vers l'intérieur le long de leurs axes longitudinaux vers l'extrémité arrière (26).
